# EUROPÄISCHE PATENTSCHRIFT

(11) **EP 1 963 483 B1**
(45) Veröffentlichungstag und Bekanntmachung des Hinweises auf die Patenterteilung: **22.05.2013**
(21) Anmeldenummer: 06805445.1
(22) Anmeldetag: 12.10.2006
(51) Int. Cl.: C12N 1/20, A61K 35/74, A23C 9/123, C12R 1/225

(54) **NEUE LACTOBACILLUS STÄMME UND DEREN VERWENDUNG GEGEN HELICOBACTER PYLORI**
NOVEL LACTOBACILLUS STRAINS AND THEIR USE AGAINST HELICOBACTER PYLORI
NOUVELLES SOUCHES DE LACTOBACILLUS ET LEUR UTILISATION CONTRE HELICOBACTER PYLORI

(30) Priorität: 22.12.2005 DE 102005062731
(43) Veröffentlichungstag der Anmeldung: 03.09.2008
(73) Patentinhaber: OrganoBalance GmbH, 13355 Berlin (DE)
(72) Erfinder: BÖTTNER, Mewes, 10407 Berlin (DE); BUDDE, Eckhard, 50733 Köln (DE); LANG, Christine, 10625 Berlin (DE); RYSER, Martin, 01326 Dresden (DE); VEEN, Markus, 84549 Engelsburg (DE)
(74) Vertreter: Jungblut, Bernhard Jakob
(86) Internationale Anmeldenummer: PCT/DE2006/001842
(87) Internationale Veröffentlichungsnummer: WO 2007/073709

(56) Entgegenhaltungen:
- WO-A-02/45727
- SERVIN A L: "Antagonistic activities of lactobacilli and bifidobacteria against microbial pathogens" FEMS MICROBIOLOGY REVIEWS, ELSEVIER, AMSTERDAM, NL, Bd. 28, Nr. 4, 1. Oktober 2004 (2004-10-01), Seiten 405-440, XP004564881 ISSN: 0168-6445
- MASTROMARINO P ET AL: "Characterization and selection of vaginal Lactobacillus strains for the preparation of vaginal tablets" JOURNAL OF APPLIED MICROBIOLOGY, OXFORD, GB, Bd. 93, 2002, Seiten 884-893, XP002995336 ISSN: 1364-5072
- KABIR A M A ET AL: "PREVENTION OF HELICOBACTER PYLORI INFECTION BY LACTOBACILLI IN A GNOTOBIOTIC MURINE MODEL", GUT, BRITISH MEDICAL ASSOCIATION, LONDON, UK, vol. 41, 1 January 1997 (1997-01-01), pages 49-55, XP000922761, ISSN: 0017-5749
- RICKARD ALEXANDER H ET AL: "Bacterial coaggregation: An integral process in the development of multi-species biofilms", TRENDS IN MICROBIOLOGY, ELSEVIER SCIENCE LTD., KIDLINGTON, GB, vol. 11, no. 2, 1 February 2003 (2003-02-01), pages 94-100, XP009154671, ISSN: 0966-842X
- AIBA Y ET AL: "Lactic acid-mediated suppression of Helicobacter pylori by the oral administration of Lactobacillus salivarius as a probiotic in a gnotobiotic murine model", AMERICAN JOURNAL OF GASTROENTEROLOGY, ELSEVIER SCIENCE INC, US, vol. 93, no. 11, 1 November 1998 (1998-11-01), pages 2097-2101, XP009154700, ISSN: 0002-9270

## Beschreibung

### Gebiet der Erfindung.

Die Erfindung betrifft neue Lactobacillus Stämme sowie deren Verwendungen, insbesondere für pharmazeutische und/oder dietätische Zusammensetzungen.

Stand der Technik und Hintergrund der Erfindung.

Probiotische Mikroorganismen umfassen lebende bzw. lebensfähige Zellen, welche in ihrer Lebendform vorteilhafte Wirkungen in menschlichen oder tierischen Körpern zeigen. Probiotische Zusammensetzungen enthalten solche Mikroorganismen. Vorteilhafte Wirkungen können insbesondere in der Verbesserung der Mikroflora des Verdauungstraktes bestehen. Insbesondere können in der Mikroflora unerwünschte andere Mikroorganismen durch unmittelbare Wechselwirkungen zwischen den probiotischen Mikroorganismen und den unerwünschten Mikroorganismen, durch mittelbare Wechselwirkungen auf Grund von Hemmungen des Metabolismus des unerwünschten Mikroorganismus durch Expressionsprodukte des probiotischen Mikroorganismus, oder durch Verstärkung des natürlichen Immunsystems gehemmt werden. Allgemein wird angenommen, dass ein Hauptmechanismus die kompetitive Besiedlung des Gastrointestinaltraktes ein wesentliches Wirkelement ist, wodurch unerwünschte Mikroorganismen die Mucosa nicht mehr in störendem Maße besiedeln können bzw. verdrängt werden.

Eine Gruppe probiotischer Mikroorganismen wird beispielsweise durch Lactobazillenstämme gebildet. Hierbei handelt es sich typischerweise um gram-positive, mikroaerophile oder anaerobe Bakterien, welche Zucker fermentieren unter Bildung von Säuren, insbesondere von Milchsäure.

Aus der Literaturstelle US-5,716,615 ist eine pharmazeutische Zusammensetzung bekannt, welche unter anderem Lactobazillen enthält. Diese ist unter anderem einsetzbar zur Behandlung von Erkrankungen des Gastrointestinaltraktes.

Aus der Literaturstelle US 2005/0186190 A1 ist eine dietätische oder pharmazeutische Zusammensetzung bekannt, welche Sphingomyelinase oder Sphingomyelinase enthaltende Lactobazillen enthält. Diese ist zur Behandlungen von Infektionen mit Heliobacter pylori geeignet.

Aus der Literaturstelle WO 2004/087891 sind Lactobacillus Stämme bekannt, welche zur Herstellungen von pharmazeutischen oder dietätischen Zusammensetzungen zur Behandlung von Infektionen des Gastrointestinaltraktes mit Helicobacter pylori geeignet sind.

Aus der Literaturstelle WO 2005/060937 A1 sind tablettenförmige Formulierungen bekannt, welche lebensfähige Lactobacillus Zellen enthalten. Diese sind zur oralen Gabe und Behandlung von Infektionen des Gastrointestinaltraktes mit Pathogenen geeignet.

Aus der Literaturstelle WO 2004/031368 A1 sind Lactobacillus Stämme bekannt, welche zur Behandlung von Entzündungen geeignet sind, die mit einer Infektion mit Helicobacter pylori assoziiert sind.

Wechselwirkungen von Lactobazillen mit Helicobacter pylori sind des weiteren bekannt aus den Literaturstellen Wang et al., Am. J. Clin. Nutr. 80:737-41 (2004), Felley et al., Best Practice & Research Clinical Gastroenterology 17(5):785-791 (2003), Cazzato et al., Scandinavian Journal of Nutrition 48(1):26-31 (2004) und Sgouras et al., Applied and Environmental Microbiology 70(1):518-526 (2004).

Aus dem Dokument WO 2002/45726 A offenbart die Verwendung von Lactobacillus fermentum Zellen zur Prophylaxe und/oder Behandlung von durch Infektion mit Helicobacter pylori bedingten Erkrankungen, insbesondere Gastrointestonalerkrankungen.

Helicobacter pylori ist eine spiralförmige Bakterie, die den Magen kolonisiert, wobei mittels Produktion von Urease der pH Wert im Magen angehoben und so die Bakterien vor der Magensäure geschützt werden. Die Bakterien penetrieren die Mucosa und lagern sich an Epithelzellen des Magens an. Eine solche Infektion aktiviert das körpereigene Immunsystem, wobei die Immunantwort jedoch nicht hinreichend effektiv zur Beseitigung der Infektion ist, mit der Folge einer sich verstärkenden Immunantwort. Letztendlich kommt es zu einer chronischen Entzündung und Erkrankung an Gastritis bzw. Magengeschwüren. Bislang ist nicht bekannt, mit welchen Mechanismen Helicobacter pylori dem Immunsystem widersteht.

Für die Wirkmechanismen der bekannten Lactobacillus Stämme gegen Helicobacter pylori werden in den vorstehend genannten Literaturstellen verschiedene Theorien unterbreitet. Gesicherte Erkenntnis über die Mechanismen bestehen jedoch noch nicht.

Insgesamt ist es wünschenswert, Lactobacillus Stämme zu entwickeln, welche die Helicobacter pylori Zellzahl im Magen seh niedrig halten und ansonsten frei von physiologischen Nebenwirkungen sind.

Technisches Problem der Erfindung.

Daher liegt der Erfindung das technische Problem zu Grunde, Lactobacillus Stämme anzugeben, die die Besiedlung der Magenmucosa mit Helicobacter pylori inhibieren.

Des weiteren liegt der Erfindung das technische Problem zu Grunde, dietätische und/oder pharmazeutische Zusammensetzungen anzugeben, die insbesondere in der Prophylaxe eine Helicobacter pylori Infektion hochwirksam sind.

Grundzüge der Erfindung und bevorzugte Ausführungsformen.

Zur Lösung dieser technischen Probleme lehrt die Erfindung die Gegenstände der Patentansprüche.

Die Erfindung beruht auf der überraschenden Erkenntnis, dass bestimmte ausgewählte Lactobacillus Stämme in der Lage sind, freie Helicobacter pylori zu binden und Aggregate zu bilden. Diese vergleichsweise großen Aggregate sind nicht mehr in der Lage, die Mucosa zu durchdringen und folglich können die Helicobacter pylori Bakterien nicht mehr die Epithelzellen des Magens erreichen und infizieren. Letztendlich wird die chronisch inflammatorische Reaktion des Immunsystems gar nicht mehr ausgelöst und eine Erkrankung an Gastritis bzw. Magengeschwüren ist zuverlässig verhindert. Die Aggregate durchlaufen den Gastrointestinaltrakt und verlassen den Körper auf natürlichem Wege. Selbst bei bereits erfolgter Infektion ist dieser Wirkmechanismus erfindungsgemäßer Lactobacillus Stämme hilfreich, da eine weitere Infektion mit zusätzlichen Helicobacter pylori Bakterien verhindert wird und so die bestehende Infektion durch Abtötung der vorhandenen Helicobacter pylori Bakterien leichter bekämpft werden kann. In der Regel gelingt dies sogar dem natürlichen Immunsystem der erkrankten Person. Hinzu kommt, dass erfindungsgemäße Lactobacillus Stämme vermutlich auch zur Hemmung der Urease Aktivität von Helicobacter pylori in,der Lage sind, so dass die Helicobacter pylori Bakterien in den Aggregaten ihren Schutz gegen den Angriff von Magensäure verlieren. Insofern wird auch ein synergistischer Effekt erzielt.

Die wesentlichen Kulturbedingungen des menschlichen Magentraktes umfassen einen pH-Wert im Bereich von 1,8 bis 4,5 und die Gegenwart von Pepsin sowie NaCl. Ein Referenzmedium, welches für solche Kulturbedingungen charakteristisch ist, besteht aus den folgenden Komponenten: Wasser, 5 g/l NaCl sowie 3 g/l Pepsin wobei der pH wert auf 2,0 mit HCl eingestellt ist.

Der Begriff der Aggregation bezeichnet die Bildung von Zellaggregaten einer Größe von zumindest 1 µm bis zu 1000 µm und mehr, enthaltend Lactobacillus Zellen und Helicobacter pylori Zellen, in Suspensionen, beispielsweise gemäß der folgenden Beispiele, insbesondere in einem Referenzmedium, wie vorstehend beschrieben.

Im Rahmen der Erfindung wurden verschiedene Lactobacillus Stämme auf Ihre Fähigkeit zur Aggregation von Helicobacter pylori untersucht und die folgenden Stämme wurden identifiziert und als erfindungsgemäße Stämme bei der DSMZ Deutsche Sammlung von Mikroorganismen und Zellkulturen GmbH, Mascheroder Weg 1b, D-38124 Braunschweig, Deutschland, hinterlegt: DSM 17646, DSM 17647, DSM 17648, DSM 17649, DSM 17650, DSM 17651, DSM 17652 und DSM 17653. Bei DSM 17646, DSM 17649, DSM 17652 und DSM 17653 handelt es sich um Lactobacillus brevis Stämme. Bei DSM 17647, DSM 17648 und DSM 17651 handelt es sich um Lactobacillus fermentum Stämme. Bei DSM 17650 handelt es sich um einen Lactobacillus pentosus Stamm.

Die Erfindung betrifft des Weiteren eine pharmazeutische und/oder dietätische Zusammensetzung enthaltend eine physiologisch wirksame Dosis an erfindungsgemäßen, vorzugsweise lebensfähigen Lactobacillus Zellen sowie einen physiologisch verträglichen Träger. Bei pharmazeutischen Zusammensetzungen handelt es sich um Zusammensetzungen, die einzig therapeutischen oder prophylaktischen Zwecken dienen und wobei neben dem Wirkstoff lediglich in der Galenik übliche Hilfs- und/oder Trägerstoffe zugegen sind. Bei dietätischen Zusammensetzungen handelt es sich um Zusammensetzungen, welche neben dem Wirkstoff auch Nahrungsmittel und Nahrungsergänzungsmittel enthalten.

Die Erfindung betrifft auch die Verwendung von erfindungsgemäßen, vorzugsweise lebensfähigen Lactobacillus Zellen zur Herstellung einer pharmazeutischen oder dietätischen Zusammensetzung, insbesondere zur Prophylaxe und/oder Behandlung von durch Infektion mit Helicobacter Pylori bedingten Erkrankungen, beispielsweise Gastrointestinalerkrankungen. Hierzu zählen insbesondere Gastritis, Magengeschwüre und Magenkrebs.

Eine erfindungsgemäße pharmazeutische Zusammensetzung kann dadurch gekennzeichnet sein, dass sie 10^2 bis 10^15, vorzugsweise 10^6 oder 10^8 bis 10^12, insbesondere 10^8 bis 10^10, Lactobacillus Zellen enthält. Bezugsgröße ist dabei eine Gabeeinheit, beispielsweise eine Tablette. Vorzugsweise ist die Zusammensetzung zur oralen Gabe hergerichtet. Die Lactobacillus Zellen werden zweckmäßigerweise lyophilisiert sein.

Die galenische Herrichtung einer erfindungsgemäßen pharmazeutischen Zusammensetzung kann in fachüblicher Weise erfolgen. Geeignete feste oder flüssige galenische Zubereitungsformen sind beispielsweise Granulate, Pulver, Dragees, Tabletten, (Mikro-) Kapseln, Suppositorien, Sirupe, Säfte, Suspensionen, oder Emulsionen, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als Hilfsstoffe sei Magnesiumcarbonat, Titandioxid, Lactose, Mannit und andere Zucker, Talcum, Milcheiweiß, Gelatine, Stärke, Zellulose und ihre Derivate, tierische und pflanzliche Öle wie Lebertran, Sonnenblumen-, Erdnuss- oder Sesamöl, Polyethylenglycole und Lösungsmittel, wie etwa steriles Wasser und ein- oder mehrwertige Alkohole, beispielsweise Glycerin, genannt. Eine erfindungsgemäße pharmazeutische Zusammensetzung ist dadurch herstellbar, dass Zellen mindestens eines erfindungsgemäß verwendeten Lactobacillus Stammes in definierter Dosis mit einem pharmazeutisch geeigneten und physiologisch verträglichen Träger und ggf. weiteren geeigneten Wirk-, Zusatz- oder Hilfsstoffen mit definierter Dosis gemischt und zu der gewünschten Darreichungsform hergerichtet ist. Als Träger kommen insbesondere Stoffe in Frage, die ausgewählt sind aus der Gruppe bestehend aus "Maltodextrin, mikrokristalline Zellulose, Stärke, insbesondere Maisstärke, Levulose, Lactose, Dextrose, und Mischungen solcher Substanzen". Die Zusammensetzung kann 0,1 bis 95 Gew.-% Träger und 5 bis 99.9 Gew.-% lyophilisierte Lactobacillus Zellen, bezogen auf die Gesamtmenge an Zellen und Träger, enthalten bzw. hieraus bestehen.

Im Falle der dietätischen Zusammensetzung kann vorgesehen sein, dass die Zusammensetzung 10^2 bis 10^15, vorzugsweise 10^6 bis 10^9, insbesondere 10^7 bis 10^9, Lactobacillus Zellen enthält. Bezugsgröße ist eine Gabeeinheit, beispielsweise eine Verpackungseinheit eines Lebensmittels zur Verkauf an einen Endverbraucher. Der physiologisch verträgliche Träger wird in der Regel ein Lebensmittel sein, welches insbesondere ausgewählt ist aus der Gruppe bestehend aus " Milchprodukte, fermentierte Milchprodukte, Milch, Yoghurt, Käse, Cerealien, Müsliriegel, und Kindernahrungszubereitungen".

Die Erfindung betrifft weiterhin ein Verfahren zur Herstellung einer erfindungsgemäßen pharmazeutischen und/oder dietätischen Zusammensetzung, wobei die lyophilisierten oder nicht lyophilisierten, vorzugsweise lebensfähigen Lactobacillus Zellen mit dem physiologisch verträglichen Träger gemischt und zur oralen Gabe hergerichtet werden.

Mit der Erfindung wird ein Verfahren zur Prophylaxe oder Behandlung einer Person ermöglicht, welche an einer durch eine Helicobacter Pylori Infektion verursachten Erkrankung, insbesondere Gastritis bzw. Magengeschwür, leidet oder hieran zu erkranken droht, wobei der Person eine physiologisch wirksame Dosis einer erfindungsgemäßen wobei der Person eine physiologisch wirksame Dosis einer erfindungsgemäßen pharmazeutischen und/oder dietätischen Zusammensetzung einbis fünfmal täglich dargereicht wird. Die Gabe kann über einen zeitlich beschränkten Zeitraum, beispielsweise 1 bis 30 Wochen, erfolgen, oder zeitlich unbeschränkt. Insbesondere letzteres eignet sich für eine dauerhafte Prophylaxe sowie eine Vorbeuge gegen Rückfallerkrankungen.

Im Folgenden wird die Erfindung anhand von lediglich Ausführungsformen darstellenden Beispielen näher erläutert.

### Beispiel 1: Lagerung verwendeter Stämme

Die Lagerung der Lactobacillus Stämme erfolgte in gefrorenem Zustand. 1 ml einer bis zur stationären Phase (OD₆₀₀/ml 4-8) in MRS Medium (55g/l, pH 6,5; Difco, USA) kultivierten Kultur wurde mit 500µl einer 50%igen (v/v) sterilen Glycerinlösung gemischt und die Mischung auf -80°C eingefroren.

Die Lagerung von Helicobacter pylori erfolgte in gefrorenem Zustand. 1 ml einer bis zur stationären Phase in Brucella broth (28g/l, pH 7,0; BD, USA), supplementiert mit 5% (v/v) defillibriertem Pferdeblut (Oxoid), kultivierten Kultur wurde mit 500µl einer 50%igen (v/v) sterilen Glycerinlösung gemischt und die Mischung auf -80°C eingefroren. Das Pferdeblut wurde vor der Verwendung gefroren und bei 20°C aufgeschlossen, um Blutzellen zu zerstören.

### Beispiel 2: Aggregation von Helicobacter pylori durch erfindungsgemäße Lactobacillus Stämme.

Die Kultivierung der Lactobazillen erfolgte in geschlossenen Falcon Röhrchen in MRS Medium bei 37°C für 24 - 48 h.

Helicobacter pylori wurde für 5 bis 6 Tage in Erlenmeyerkolben unter mikroaerophilen Bedingungen und ansonsten wie in Beispiel 1 beschrieben kultiviert.

Nach der Kultivierung wurde die Zellmorphologie mikroskopisch untersucht. Es wurden Assays durchgeführt mit Kulturen bestehend sowohl aus Zellen mit sigmoidaler Morphologie als auch aus Zellen mit coccoider Morphologie. Auch Kulturen mit gemischter Morphologie wurden untersucht.

Die jeweiligen Zellen wurden durch Zentrifugation bei 3200 g für 10 min. geerntet und der Überstand wurde verworfen. Die Zellen wurden einmal in 5 ml Puffer gewaschen und in 5 ml Puffer resuspendiert (PBS-Puffer enthaltend 1,5 5 g/l Na₂HPO₄*2H₂O, 0,2 g/l KH₂PO₄ und 8,8 g/l NaCl). Der pH Wert wurde mit HCl auf 7,0 eingestellt. Der OD₆₀₀ Wert wurde gemessen und auf einen Wert von 2 durch Zugabe von Puffer eingestellt.

2,5 ml jeder so erhaltenen Zellsuspension (Heliobacter pylori/Lactobacillus) wurden gemischt und die Mischung wurde für 10 min. gevortext. Das Ergebnis wurde mikroskopisch untersucht. Kontrollexperimente auf Selbstaggregation wurden durch separate Untersuchung von Kulturen mit jeweils Lactobacillus und Helicobacter pylori allein durchgeführt.

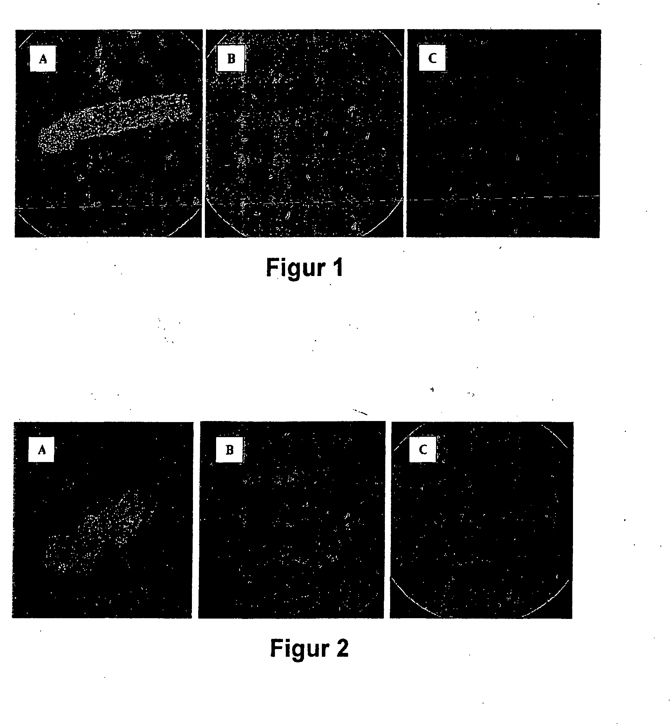

In der Figur 1 erkennt man, dass sich in der Suspension mit einer Mischung von Lactobacillus und Helicobacter pylori große Aggregate gebildet haben, während solche Aggregate in den Kontrollexperimenten abwesend sind. Dieses Ergebnis wird für alle erfindungsgemäßen Stämme erhalten, in der Figur 1 ist lediglich beispielhaft der Lactobacillus Stamm DSM 17648 dargestellt.

Fig. 1A zeigt ein typisches Aggregat von Helicobacter pylori durch den Stamm DSM 17647. Fig. 1B zeigt den Stamm DSM 17648 alleine. Fig. 1C zeigt Helicobacter pylori alleine. Die Vergrößerung ist 1000-fach. Die Aggregate haben typischerweise eine Größe von 1 µm, in der Regel von 5 µm bis zu 50 µm, oder, wie in Fig. 1A, sogar bis zu 1000 µm und mehr (größte Erstreckung).

Grundsätzlich lassen sich in Frage kommende Lactobacillus Stämme mit einem solchen Aggregationstest dahingehend untersuchen, ob der untersuchte Stamm eine solche Aggregation aus Lactobacillus und Helicobacter pylori induziert.

### Beispiel 3: Simulation der Bedingungen des Magentraktes.

Zur Simulation der in vivo Verhältnisse wurden die Experimente des Beispiels 2 wiederholt, wobei die Resuspension der Lactobacillus Zellen jedoch in simuliertem Magensaft (5g/l NaCl und 3 g/l Pepsin (Sigma)) erfolgte. Der pH wurde mit HCl auf 2 eingestellt. Diese Inkubation erfolgte für 30 min. bei 37 °C. Da Helicobacter pylori den pH-Wert in unmittelbarer Umgebung der Zellen auf pH 4 anheben kann, wurden die Zellen, wie in Beispiel 2 beschrieben geerntet und dann in Acetatpuffer pH 4 resuspendiert. Zur Einstellung des Puffers wurden 41 ml einer Lösung mit 0,1 Mol/l Essigsäure mit einer Lösung mit 0,2 Mol/l Natriumacetat auf pH 4 eingestellt. Mit Wasser wurde auf ein Endvolumen von 100 ml aufgefüllt.

Die Helicobacter pylori Zellen wurden entsprechend Beispiel 2 kultiviert.

Nach der Ernte der Zellen entsprechend dem Beispiel 2 wurden die Zellen abweichend vom Beispiel 2 in Acetatpuffer (s.o.) resuspendiert. Hiernach wurden die Aggregationsversuche entsprechend Beispiel 2 ausgeführt. Die Ergebnisse sind in der Figur 2 dargestellt. Fig. 2A zeigt ein typisches Aggregat von Helicobacter pylori durch den Stamm DSM 17648 nach einer simulierten Magenpassage. Fig. 2B zeigt Stamm DSM 17648 alleine nach einer simulierten Magenpassage. Fig. 2C zeigt Helicobacter pylori alleine. Die Vergrößerung ist 1000-fach. Man erkennt, dass die Größe der in der Mischung erhaltenen Aggregate im Bereich von 2 µm bis 1000 µm und mehr liegt.

Auch diese Variante eines Aggregationsversuches ist geeignet, erfindungsgemäße Lactobacillus Stämme zu identifizieren.

### Beispiel 4: Effekt der Lyophilisierung von Lactobacillus

Die Bakterien wurden entsprechend Beispiel 1 gezogen. Aliquoten von 1 ml der Lactobacillus Kulturen wurden durch Zentrifugation bei 3200 g für 10 min. geerntet. Der Überstand wurde verworfen und die Pellets wurden für 2 h unter Vakuum lyophilisiert. So erhaltene getrocknete Pellets jedes der erfindungsgemäßen Lactobacillus Stämme wurden in 1 ml PBS Puffer, pH 7,0, resuspendiert. Die resuspendierten Lactobacillus Zellen wurden in einem Volumenverhältnis von 1:1 mit frisch gezogenen Helicobacter pylori Kulturen gemischt und die Aggregation wurde wie in den Beispielen 2 und 3 bestimmt. Die Fähigkeit der Lactobacillus Zellen, eine Aggregation von Helicobacter pylori zu induzieren, wurde durch die Lyophilisierung nicht beeinflusst, wie Untersuchungen entsprechend der vorhergehenden Beispiele zeigte (Eine fotographische Dokumentation erfolgte jedoch nicht).

### Beispiel 5: Bestimmung der Species

Die taxonomische Bestimmung der erfindungsgemäßen Lactobacillus Stämme erfolgte anhand deren Kohlenhydratfermentierungsmuster. Dies wurde bestimmt unter Verwendung des API 50 CH Systems (bioMerieux, Frankreich) und die Analyse erfolgte mit der APILAB PLUS Software (Release 3.3.3 des gleichen Herstellers). Die Bestimmung erfolgte nach Herstellervorgaben.

### Beispiel 6: Herstellung einer pharmazeutischen Zusammensetzung mit erfindungsgemäßen Lactobacillus Stämmen.

Zellen eines Lactobacillus Stammes oder mehrerer Lactobacillus Stämme der Erfindung werden gemäß Beispiel 4 gezogen und lyophilisiert. Das Pellet wird dann auf eine Partikelgröße von maximal ca. 1 mm Durchmesser gemahlen. Das erhaltene Granulat wird in den folgenden Mengenverhältnissen (Gew.-%) mit Träger- bzw. Hilfsstoffen gemischt:
20% Granulat
2% Siliciumdioxid (Syloid AL-1FP, GRACE Davidson)
1% Magnesiumstearat (MF-2-V, Ackros)
77% mikrokristalline Zellulose (Avicel PH 112, FMC)

Das Mischen erfolgt in einem Quintech Micromixer bei Position 70 Level II. Alle Komponenten werden zugleich zugegeben. Die Mischung erfolgt für ca. 120 s. Anschließend wird die erhaltene Mischung in einer handelsüblichen Tablettenpresse unter üblichen Bedingungen, jedoch möglich niedriger Druckkraft (< 10 kN) zu Tabletten mit einem Gewicht von ca. 500 mg gepresst. Jede Tablette enthält ca. 10^8 bis 10^10 Lactobacillus Zellen.

### Beispiel 7: Herstellung einer dietätischen Zusammensetzung mit erfindungsgemäßen Lactobacillus Stämmen.

Zellen eines Lactobacillus Stammes oder mehrerer Lactobacillus Stämme der Erfindung werden gemäß Beispiel 4 gezogen und lyophilisiert. Lyophilisat enthaltend ca. 10^7 bis 10^8 Lactobacillus Zellen wird mit jeweils ca. 1 1 handelüblicher pateurisierter Milch vermischt und bei 5 °C kurz homogenisiert. Die homogenisierte Milch wird dann in üblicher Weise abgefüllt und verpackt.

## Patentansprüche

1. Verwendung von Lactobacillus Zellen wie hinterlegt unter DSM 17646, DSM 17647, DSM 17648, DSM 17649, DSM 17650, DSM 17651, DSM 17652 oder DSM 17653, zur Herstellung einer pharmazeutischen oder dietätischen Zusammensetzung zur Prophylaxe und/oder Behandlung von durch Infektion mit Helicobacter pylori bedingten Erkrankungen, insbesondere Gastrointestinalerkrankungen, wie Magengeschwür, wobei die Lactobacillus Zellen in der Lage sind, Helicobacter pylori unter Kulturbedingungen des menschlichen Verdauungstraktes, insbesondere des Magens, zu aggregieren.

2. Verwendung nach Anspruch 1, wobei die pharmazeutische Zusammensetzung 10^2 bis 10^15, vorzugsweise 10^6 oder 10^8 bis 10^12, insbesondere 10^8 bis 10^10, Lactobacillus Zellen enthält.

3. Verwendung nach einem der Ansprüche 1 bis 2, wobei die pharmazeutische Zusammensetzung zur oralen Gabe hergerichtet ist.

4. Verwendung nach einem der Ansprüche 1 bis 3, wobei die Lactobacillus Zellen in der pharmazeutischen Zusammensetzung lyophilisiert sind.

5. Verwendung nach einem der Ansprüche 1 bis 4, wobei der Träger der pharmazeutischen Zusammensetzung ausgewählt ist aus der Gruppe bestehend aus "Maltodextrin, mikrokristalline Zellulose, Stärke, insbesondere Maisstärke, Levulose, Lactose, Dextrose, und Mischungen solcher Substanzen".

6. Verwendung nach einem der Ansprüche 1 bis 5, wobei die pharmazeutische Zusammensetzung 0,1 bis 95 Gew.-% Träger und 5 bis 99.9 Gew.-% lyophilisierte Lactobacillus Zellen enthält.

7. Verwendung nach Anspruch 1, wobei die dietätische Zusammensetzung 10^2 bis 10^15, vorzugsweise 10^6 bis 10^9, insbesondere 10^7 bis 10^9, Lactobacillus Zellen enthält.

8. Verwendung einem der Ansprüche 1 oder 8, wobei der physiologisch verträgliche Träger der dietätischen Zusammensetzung ein Lebensmittel ist, insbesondere ausgewählt ist aus der Gruppe bestehend aus "Milchprodukte, fermentierte Milchprodukte, Milch, Yoghurt, Käse, Cerealien, Müsliriegel, und Kindernahrungszubereitungen".

9. Verfahren zur Herstellung einer pharmazeutischen und/oder dietätischen Zusammensetzung nach einem der Ansprüche 1 bis 9, wobei eine physiologisch wirksame Menge der lyophilisierten oder nicht lyophilisierten Lactobacillus Zellen mit dem physiologisch verträglichen Träger gemischt und zur oralen Gabe hergerichtet werden.

10. Isolierte Lactobacillus Zellen, welche in der Lage sind, Helicobacter pylorei unter Kulturbedingungen des manschlichen Verdauungstraktes zu aggregieren, nämlich wie hinterlegt unter DSM 17646, DSM 17647, DSM 17648, DSM 17649, DSM 17650, DSM 17651, DSM 17652 oder DSM 17653.

11. Pharmazeutische und/oder dietätische Zusammensetzung enthaltend eine physiologisch wirksame Dosis an Lactobacillus Zellen nach Anspruch 10 sowie einen physiologisch verträglichen Träger.

12. Verwendung von Lactobacillus Zellen nach Anspruch 10 zur Herstellung einer pharmazeutischen oder dietätischen Zusammensetzung.

## Claims

1. A use of Lactobacillus cells as filed under DSM 17646, DSM 17647, DSM 17648, DSM 17649, DSM 17650, DSM 17651, DSM 17652 or DSM 17653, for producing a pharmaceutical or dietetic composition for the prophylaxis and/or treatment of diseases caused by infection with Helicobacter pylori, in particular gastrointestinal diseases, such as stomach ulcer, wherein the Lactobacillus cells are capable of aggregating Helicobacter pylori under culture conditions of the human digestive tract, in particular of the stomach.

2. The use according to claim 1, wherein the pharmaceutical composition contains 10^2 to 10^15, preferably 10^6 or 10^8 to 10^12, in particular 10^8 to 10^10, Lactobacillus.cells.

3. The use according to one of claims 1 to 2, wherein the pharmaceutical composition is prepared for oral administration.

4. The use according to one of claims 1 to 3, wherein the Lactobacillus cells in the pharmaceutical composition are lyophilized.

5. The use according to one of claims 1 to 4, wherein the carrier of the pharmaceutical composition is selected from the group comprising "maltodextrin, microcrystalline cellulose, starch, in particular corn starch, levulose, lactose, dextrose, and mixtures of such substances".

6. The use according to one of claims 1 to 5, wherein the pharmaceutical composition contains 0.1 to 95% by weight carrier and 5 to 99.9% by weight lyophilized Lactobacillus cells.

7. The use according to claim 1, wherein the dietetic composition contains 10^2 to 10^15, preferably 10^6 to 10^9, in particular 10^7 to 10^9, Lactobacillus cells.

8. The use according to one of claims 1 or 8, wherein the physiologically tolerated carrier of the dietetic composition is a food material, in particular selected from the group comprising "milk products, fermented milk products, milk, yogurt, cheese, cereals, muesli bars, and children's food preparations".

9. A method for producing a pharmaceutical and/or dietetic composition according to one of claims 1 to 9, wherein a physiologically effective quantity of the lyophilized or non-lyophilized Lactobacillus cells is mixed with the physiologically tolerated carrier and prepared for oral administration.

10. Isolated Lactobacillus cells being capable of aggregating Helicobacter pylori under culture conditions of the human digestive tract, namely as filed under DSM 17646, DSM 17647, DSM 17648, DSM 17649, DSM 17650, DSM 17651, DSM 17652 or DSM 17653.

11. A pharmaceutical and/or dietetic composition containing a physiologically effective dose of Lactobacillus cells according to claim 10 and a physiologically tolerated carrier.

12. The use of Lactobacillus cells according to claim 10 for producing a pharmaceutical or dietetic composition.

## Revendications

1. Utilisation de cellules Lactobacillus comme déposées sous DSM 17646, DSM 17647, DSM 17648, DSM 17649, DSM 17650, DSM 17651, DSM 17652 ou DSM 17653, pour la production d'une composition pharmaceutique ou diététique pour la prophylaxie et/ou le traitement de maladies dues à une infection avec Helicobacter pylori, en particulier maladies gastro-intestinales, comme p.ex. ulcère à l'estomac, dans laquelle les cellules Lactobacillus sont aptes à agréger Helicobacter pylori sous des conditions de culture du tractus digestif humain, en particulier de l'estomac.

2. Utilisation selon la revendication 1, dans laquelle la composition pharmaceutique contient 10^2 à 10^15, de préférence 10^6 ou 10^8 à 10^12, en particulier 10^8 à 10^10, cellules Lactobacillus.

3. Utilisation selon une des revendications 1 à 2, dans laquelle la composition pharmaceutique est préparée pour l'administration orale.

4. Utilisation selon une des revendications 1 à 3, dans laquelle les cellules Lactobacillus dans la composition pharmaceutique sont lyophilisées.

5. Utilisation selon une des revendications 1 à 4, dans laquelle le porteur de la composition pharmaceutique est choisi à partir du groupe comprenant «maltodextrine, cellulose microcristalline, amidon, en particulier amidon de maïs, levulose, lactose, dextrose, et mélanges de telles substances».

6. Utilisation selon une des revendications 1 à 5, dans laquelle la composition pharmaceutique contient 0,1 à 95% en poids de porteur et 5 à 99.9% en poids de cellules Lactobacillus lyophilisées.

7. Utilisation selon la revendication 1, dans laquelle la composition diététique contient 10^2 à 10^15, de préférence 10^6 à 10^9, en particulier 10^7 à 10^9, cellules Lactobacillus.

8. Utilisation selon une des revendications 1 ou 8, dans laquelle le porteur physiologiquement acceptable de la composition diététique est un produit alimentaire, en particulier choisi à partir du groupe comprenant «produits laitiers, produits laitiers fermentés, lait, yaourt, fromage, céréales, barres de muesli, et préparations d'aliments premier âge».

9. Procédé de production d'une composition pharmaceutique et/ou diététique selon une des revendications 1 à 9, dans lequel une quantité physiologiquement efficace des cellules Lactobacillus lyophilisées ou non lyophilisées est mélangée avec le porteur physiologiquement acceptable et préparée pour l'administration orale.

10. Cellules Lactobacillus isolées étant aptes à agréger Helicobacter pylori sous des conditions de culture du tractus digestif humain, c'est-à-dire comme déposées sous DSM 17646, DSM 17647, DSM 17648, DSM 17649, DSM 17650, DSM 17651, DSM 17652 ou DSM 17653.

11. Composition pharmaceutique et/ou diététique contenant une dose physiologiquement efficace de cellules Lactobacillus selon la revendication 10 et un porteur physiologiquement acceptable.

12. Utilisation de cellules Lactobacillus selon la revendication 10 pour la production d'une composition pharmaceutique ou diététique.
